# EUROPEAN PATENT SPECIFICATION

(11) **EP 4 114 474 B1**
(45) Date of publication and mention of the grant of the patent: **18.06.2025**
(21) Application number: 21725244.4
(22) Date of filing: 05.03.2021
(51) Int. Cl.: A61L 15/20, A61L 15/44, A61L 24/00, A61L 26/00

(54) **HEMOSTATIC AGENT**
HÄMOSTATISCHES MITTEL
AGENT HÉMOSTATIQUE

(30) Priority: 05.03.2020 IT 202000004642
(43) Date of publication of application: 11.01.2023
(73) Proprietor: Industria Farmaceutica Nova Argentia S.r.l., 20064 Gorgonzola (IT)
(72) Inventor: RIVA, Danilo, 22033 Asso (IT); PUMILIA, Gloria, 20125 Milano (IT); LEROSE, Alice, 20099 Sesto San Giovanni (IT); BONERI, Marco, 20882 Bellusco (IT)
(74) Representative: Valenza, Silvia
(86) International application number: PCT/IB2021/051851
(87) International publication number: WO 2021/176410

(56) References cited:
- EP-A1- 2 521 538
- WO-A2-2004/043343
- GB-A- 594 185
- US-A- 4 395 398
- US-A1- 2002 088 590
- US-A1- 2005 277 088
- US-A1- 2009 148 502
- US-A1- 2019 352 183
- HE YIN-FENG ET AL: "Iron Overload Inhibits Osteoblast Biological Activity Through Oxidative Stress", BIOLOGICAL TRACE ELEMENT RESEARCH, HUMANA PRESS, CLIFTON, NJ, US, vol. 152, no. 2, 19 January 2013 (2013-01-19), pages 292 - 296, XP035319121, ISSN: 0163-4984, [retrieved on 20130119], DOI: 10.1007/S12011-013-9605-Z
- YAMASAKI K ET AL: "Excess iron inhibits osteoblast metabolism", TOXICOLOGY LETTERS, ELSEVIER BIOMEDICAL PRESS, AMSTERDAM, NL, vol. 191, no. 2-3, 15 December 2009 (2009-12-15), pages 211 - 215, XP026764544, ISSN: 0378-4274, [retrieved on 20090906], DOI: 10.1016/J.TOXLET.2009.08.023

## Description

### FIELD OF THE INVENTION

The present invention relates to the field of hemostatic agents, in particular to a hemostatic agent based on ferric and aluminum salts.

### BACKGROUND

Hemostasis is the arrest of a bleeding and therefore is configured as an act of paramount importance for the patient. Currently there are several drugs and topical devices, designed to reduce blood loss during bleeding, such as the one due to a simple epistaxis or surgery; the main products are hemostatics, adhesives and sealants.

Hemostatic agents facilitate or accelerate the physiological hemostatic process through: reproduction of the final phase of the common pathway of coagulation; platelet adhesion and activation; concentration of the blood corpuscle.

Choosing the most appropriate hemostatic agent requires careful consideration of two main aspects: site and type of bleeding, as well as product characteristics.

All of them must be safe, effective, easy to use and inexpensive.

In relation to the origin of the components, hemostatic agents can be classified into: topical resorbable hemostats, blood derivatives and surgical glues.

There are medical devices based on collagen or jellies that stop oozing bleeding and activate the coagulation process through a chemical and/or mechanical action. There are also hemostatic blood derivatives: they are drugs that require appropriate use, monitoring and pharmacovigilance; they block major bleeding as they simulate the last step of the coagulation cascade and also have a sealing action on the surfaces of the tissues where they are applied.

There are products based on calcium alginate: the latter promotes the aggregation of platelets by reducing the clotting time, as it releases calcium particles that are exchanged with sodium particles in the blood (calcium alginate gels, creating a humid environment conducive to healing).

The aluminium potassium sulfate dodecahydrateor potassium-alum better known as potassium alum and rock alum is a mixed salt of aluminum and potassium of sulfuric acid and it appears as a colourless and odourless solid at room temperature. Since ancient times it was used to wash wounds, in numerous productive activities and in various sectors. It was then used for the production of glass and it was used as a hemostatic in medicine. The production of alum took place by heating and subsequent dissolution of alunite in water, a basic potassium and aluminum sulphate found in nature. The term "alum" refers to double sulphates of a monovalent metal and of a trivalent metal (e.g., aluminum or chromium, vicariant ion thereof) dodecahydrates. Alum was already mined at the time of the Romans and was exported in amphorae that have been found as far as England. This is because the extracted alum was a rare and sought-after substance; a very powerful hemostatic, as well as excellent for tanning leather.

For the treatment of superficial wounds, in particular bleeding phenomena due to epistaxis, in adults and paediatric subjects, there are hemostatic cottons based on calcium alginates and also based on FeCl₃ (marketed by the same Applicant). Hemostatic cotton soaked in FeCl₃ has proved particularly effective in stopping superficial bleeding, however it has shown a series of problems both in the production and storage phases. FeCl₃ is a very soluble salt in water; it is dissociated in the following way:

FeCl₃(s) → Fe³⁺(aq) + 3 Cl⁻(aq)

However, diluted solutions of ferric chloride hydrolyze over time: at ordinary temperature, dissociation leads to the formation of a basic chloride, according to the chemical equilibrium:

FeCl₃ + H₂O ↔Fe(OH)Cl₂ + HCl

The production of HYDROCHLORIC ACID (HCl) leads to damage to the production machinery as it is extremely corrosive (with consequently the need to replace some iron parts at each production) and also leads to a reduced stability of the finished product: the cotton wool flakes off. It maintains the hemostatic properties but makes the product less stable, inducing a more rapid disintegration thereof. US 2005/0277088 A1 discloses gingival retraction cords comprising cotton and a hemostatic agent absorbed therein. The hemostatic agent is selected from potassium aluminum sulfate, aluminum ammonium sulfate, aluminum sulfate, aluminum chlorhydrate, aluminum acetate, ferric sulfate, ferric subsulfate, ferric chloride, and mixtures thereof.

The object of the present invention is to provide a hemostatic agent, which maintains the same hemostatic capacity observed for FeCl₃ but without the problems associated with the generation of HCl.

### SUMMARY OF THE INVENTION

The present invention solves the aforesaid problems by means of a hemostatic agent comprising ferric ammonium citrate (FAC) and aluminum potassium sulphate dodecahydrate (potassium alum), wherein ferric ammonium citrate (FAC) is brown or red.

Surprisingly, the presence of exactly those Fe³⁺ and Al³⁺ salts was found suitable to promote the coagulation of blood protein fractions. Alongside this primary mechanism, a parallel stimulation of Fibrinogen I by ferric ions is postulated, which however are present in Iron(III) Ammonium Citrate in chelated form.

The object of the present invention is also a hemostatic composition adapted for topical administration comprising the hemostatic agent of the invention.

The object of the present invention is also a hemostatic cotton consisting of carded cotton comprising the hemostatic agent of the invention.

In the device object of the present invention, surprisingly, the action of the mechanical type, packing of cuts and wounds, given by the cotton is assisted by the presence of Fe³⁺ and Al³⁺ ions.

The presence of ferric ammonium citrate results in the following advantages:
- It does not release hydrochloric acid either at ambient temperature or in heat and therefore it is non-corrosive for machinery or cotton fibres;
- It has a very high water solubility > 1000 g/l, forming neutral aqueous solutions (i.e., at about pH 7)
- It maintains a hemostatic activity similar to FeCl₃ at the same concentration of ferric ions (preferably 15-35 g Fe/100 g of hemostatic cotton; more preferably 21 g Fe/100 g of hemostatic cotton)

The hemostatic agent of the invention is useful for medical use in the treatment of bleeding phenomena. The composition according to the present invention is particularly useful for use in the treatment of superficial wounds, surgical wounds, ulcers, sores or other skin lesions. The hemostatic cotton according to the present invention is particularly useful for use in the treatment of superficial wounds, and more particularly bleeding phenomena due to epistaxis, in adults and paediatric subjects.

The object of the present invention is also a continuous process for the preparation of the above mentioned hemostatic cotton, said process comprising:
preparation of a wetting aqueous solution comprising iron(III) ammonium citrate and double sulphate of aluminum and potassium dodecahydrate;
imbibition of a cotton flap with the wetting solution;
drying.

Advantageously, the process of the present invention does not lead to corrosion of the metal parts of the machinery and has a more efficient drying system than the production process used for the state-of-the-art hemostatic cotton comprising FeCl3 which was prepared with a similar method.

### DETAILED DESCRIPTION OF THE INVENTION

For the purposes of the present invention, bleeding phenomena are understood to mean bleeding of any type including those deriving from more or less superficial surgical operations.

For the purposes of the present invention, ferric ammonium citrate (FAC) is brown or red.

In the hemostatic agent object of the present invention the Fe(III) salt is present in a proportion by weight of 4:1-2:1 with respect to the Al(III) salt, preferably in a proportion of 3:1.

A hemostatic composition according to the present invention is in a form adapted for topical application, for example and preferably in the form of powder, spray solution, gel, ointment or cream, or gauze or plaster.

This administration can be carried out using different substrates, namely:
Carded cotton wool
Water-wetted cotton wool
Gauze, Plasters
Colloidal silica

Furthermore, the hemostatic composition can also be employed in a gel formulation. The hemostatic composition according to the present invention, in addition to the usual excipients or support materials, can also comprise one or more ingredients selected from arginine, a modulator of Filaggrin and/or Fibronectin and/or Elastin, aloe, Silver ions, hyaluronic acid and Tamarindus Indica. Said modulator of Filaggrin and/or Fibronectin and/or Elastin is preferably a low molecular weight oligopeptide from pure rice proteins (commercially available as Integrine-60 from Vevy Europe S.p.A.).

In the case of a powder formulation, it preferably comprises as excipient/substrate hydrophobic colloidal silica (preferably Silanamine, 1,1,1-trimethyl-N-(trimethylsinyl) hydrolysis products; commercially available as AEROSIL^{®} R812S).

A surprisingly effective coagulant activity in accidental or surgical wounds, even deep ones, can be obtained with a hemostatic powder according to the present invention comprising the hemostatic agent of the invention and hydrophobic colloidal silica. Preferably the hemostatic powder of the present invention comprises:
FAC 4-6% w/w; more preferably 5.0-5.5% w/w;
alum 1-2% w/w; more preferably 1.5-2.0% w/w;
silica 2-8% w/w; more preferably 5-7% w/w;
arginine 0-5% w/w; if present, more preferably 1-5% w/w;
Integrine-60 0-5% w/w; if present more preferably 1.5-2.0% w/w
purified water 100%.

The aforesaid % w/w are meant with respect to the weight of the purified water. Hemostatic powder is preferably indicated for the treatment of abrasions, superficial wounds, surgical wounds, bedsores, varicose or diabetic ulcers.

Hemostatic powder was surprisingly effective allowing for example the complete and practically immediate and complete healing of superficial wounds, or allowing the healing of bedsores, ulcers or diabetic foot that are otherwise incurable with surprisingly shorter times than other hemostatic or healing remedies known to the state of art.

Also disclosed, but not part of the present invention, a process for the preparation of a hemostatic powder is described, said process comprising:
adding, under continuous stirring, one at a time and after complete dissolution, the components FAC, alum and optionally arginine and integrine-60;
finally adding silica always under stirring, preferably vigorously.

Depending on the indication of use, the composition of the hemostatic powder of the invention can be modulated above all as regards the presence and content of arginine and of the modulator of Filaggrin and/or Fibronectin and/or Elastin.

An effective coagulant activity in epistaxis can be obtained with a hemostatic cotton according to the present invention comprising 21% ± 6% fi Fe(III), preferably 21% ± 3.5%, of Fe(III), while that for superficial abrasions due to rubbing can be obtained with a hemostatic cotton, a hemostatic gauze or a hemostatic composition according to the present invention comprising 8% ± 2% of Fe(III); where % refer to percentages by weight with respect to each 100 g of finished product.

The hemostatic composition of the invention preferably comprises 5-10 g of Fe/100g of composition; more preferably 6-10 g of Fe/100g of composition.

The hemostatic cotton of the invention for epistaxis preferably comprises 17-25 g of Fe/100 g, more preferably 19-23 g of Fe/100 g.

The hemostatic cotton according to the present invention is preferably a hemostatic cotton consisting of a carded (and bleached) cotton wool and/or a water-treated cotton wool soaked with an aqueous solution of ferric ammonium citrate and double sulphate of aluminum and potassium dodecahydrate and then dried.

According to the process object of the present invention, the wetting solution is an aqueous solution of ferric ammonium citrate and double sulphate of aluminum and potassium dodecahydrate (i.e., an aqueous solution in which only these two salts are present). The wetting solution preferably has a concentration by weight of FAC at 5.0-5.5% w/v and Rock Alum 1.5-2.0% w/v, more preferably FAC 5.3% w/v and Rock Alum 1.7% w/v.

The imbibition takes place by immersing a cotton flap in the wetting solution so that the flap is completely immersed. The imbibition takes place preferably at ambient temperature and for a maximum time of 1 minute. The soaking time can vary depending on the ambient temperature and pressure.

The drying step preferably comprises first pressing and then hot drying. Therefore preferably, after soaking, the excess wetting solution is removed by pressing the soaked cotton flap between two press rollers. Depending on the soaking power of the flap and the Salt residue to be obtained on the dry product, the pressure of the press is adjusted manually by the operator by distancing or bringing the rollers closer. After pressing, a hot drying phase follows at a temperature of 55 ± 5°C for 45-90 min or at a temperature of 50 ± 5°C for 1-2 hrs followed by a temperature of 40 ± 5°C for 0.5 -1.5 hrs in turn followed by a temperature of 30 ± 5°C for at least 7

hrs and in any case until completely dry. Depending on the temperature and humidity, the drying time can preferably vary from 50 min to 85 min. The drying time of the FeCl3-based hemostatic cotton required 120 min, produced in a similar manner.

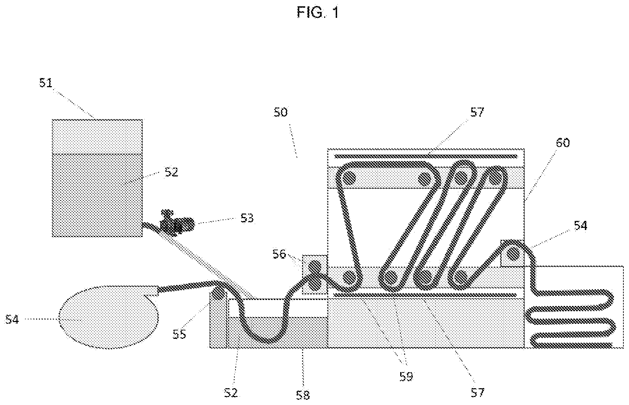

With reference to fig. 1, not according to the present invention is also a machinery (50) specifically suitable for carrying out the continuous process referred to above, said machinery comprising:
a container (51) suitable for the preparation of a wetting solution (52);
an imbibition tank (58) in fluid connection with the container (51) in which the flow of wetting solution from the container to the tank is managed by a pumping means (53);
a roller handling system (55) to move a cotton flap (54) and allow it to enter the imbibition tank (58);
a wringing system (56) above the imbibition tank, said wringing system in line with the handling system so as to receive and move the flap (54) exiting the imbibition tank (58);
a conveyor belt (59) adjacent to the imbibition tank and in line with the wringing system (56) so as to be able to receive the soaked material leaving the wringing system; said conveyor belt adapted to transport and keep the soaked material in a drying area (60) equipped with heating means (57) for a sufficient time to dry the soaked and wrung material.

In an alternative embodiment of the machinery of the invention, the conveyor belt, with a path parallel to the floor, does not have a drying area and at the exit from the conveyor belt the cotton is portioned and positioned in trays and subjected to drying in a static oven.

The cotton flap can be several thousand metres long and therefore the process of the invention takes place continuously with gradual unrolling of the flap. The process for soaking and drying an entire cotton flap about 2 km long can take about 3 months.

The heating means (57) are preferably electrical resistors.

The container (51) is provided with stirring means, preferably a magnetic plate and a stir bar or with a lance stirrer.

The process described herein for the production of a hemostatic cotton according to the present invention can be applied, mutatis mutandis, for the production of a hemostatic gauze.

The present invention can be better understood in the light of the following embodiment examples.

### BRIEF DESCRIPTION OF THE FIGURES

Figure 1 shows the machinery according to the present invention.
Fig. 2 shows the *in vitro* cytotoxic effect on murine fibroblasts (A): Control - CTR; (B): Iron chloride 5% - FeCl3; (C); Iron ammonium citrate 5.3% + Rock Alum 1.7% - hemostatic mixture of the invention (NovaStop); (D) Iron ammonium citrate 5.3% - Iron amm. citr.; (E): Rock Alum 1.7% - Rock Alum

### EXPERIMENTAL PART

### EXAMPLE 1 - PRODUCTION SCHEME

### A)

### EQUIPMENT

Polythene container
Lance stirrer
Soaking machine provided with an imbibition tank, a pump suitable for filling said tank and two press rollers.
Drying oven.

### ENVIRONMENTAL PARAMETERS

Temperature (THD32): 22°C ± 3°C
Humidity (THD32): RH%: ≤ 65 %
Pressure (PI-16): ΔP: 10 - 35 Pa

### PRODUCTION PROCESS

### Weighing of raw materials

| Component | Amount (kg) |
|---|---|
| Ferric ammonium citrate (brown) | 6.36 |
| KAl(SO₄)₂·12H₂O | 2.04 |
| Purified water | 120 |
| 100% carded cotton wool | 10.00 |

The cotton comes in the form of flaps of about 2000 m in length; 360 mm ± 3 mm of width 220 g/m² ± 10% by weight. It is 100% cotton bleached with hydrogen peroxide.

### Preparation of the wetting solution

In a polythene container, dissolve the ferric salt and the aluminum salt in purified Water while stirring.

Stir with the lance stirrer for about 20 minutes and check for complete dissolution.

### Imbibition

Connect the drum containing the wetting solution to the pump of the soaking machine which keeps the liquid level in the imbibition tank constant.

Insert the cotton flap in the imbibition tank so that it is completely immersed. Allow to soak for 3-6 min.

### Drying

Slide the cotton flap through the two press rollers positioned at a distance of 1.5 mm. ± 07 mm.

Place the pressed flap on the mat of the drying oven and allow the cotton dry at 55°C± 5°C for 50-85 min depending on the humidity and ambient temperature conditions.

The final product has a content of Fe(III) ions equal to 21% ± 6%, preferably 21% ± 3.5%, by weight for each 100 g of hemostatic cotton.

### B)

### EQUIPMENT

Polythene container
Magnetic stirrer with stir bar
Soaking machine provided with an imbibition tank, a pump suitable for filling said tank and two press rollers.
Drying oven.

### ENVIRONMENTAL PARAMETERS

Temperature (THD32): 22°C ± 3°C
Humidity (THD32): RH%: ≤ 65 %
Pressure (PI-16): ΔP: 10 - 35 Pa

### PRODUCTION PROCESS

| Component | Unit quantity (g) | Batch Quantity (kg) | Batch Quantity (kg) |
|---|---|---|---|
| 100% CARDED COTTON WOOL | 1.298 | 10.000 | |
| COTTON WOOL | 1.298 | | 5.900 |
| Solution | | | |
| FAC | 0.910 | 4.239 | 4.239 |
| ROCK ALUM | 0.292 | 1.356 | 1.356 |
| PURIFIED WATER | 17.170 | 80.000 | 80.000 |

The types of cotton used have the following characteristics:
100% carded cotton wool:
   - Material: 100% cotton bleached with hydrogen peroxide (contains no optical bleaches)
   - Absorption coefficient 23 g x g
   - Width 500 mm ± 10%
   - Weight: 400 g/m² / gsm ± 10%
Cotton wool
   - Material: 100% cotton bleached with hydrogen peroxide (contains no optical bleaches)
   - Absorption coefficient 23 g x g
   - Width: 360 mm (+/- 3 mm)
   - Weight: 220 g/m² ± 10%

### Preparation of the wetting solution

In a polythene container add purified water, operate the stirrer and the speed of the stir bar at 130 rpm; slowly add the ferric salt and increase the speed of the stir bar up to 550rpm. Decrease the speed up to 300rpm and keep it for 10-15 min or until the raw material is completely dissolved. Reduce the speed of the stir bar to 180rpm and slowly add the rock alum; increase the speed up to 30rpm and keep it for 15 minutes until the raw material is completely dissolved.

### Imbibition

Connect the drum containing the wetting solution to the pump of the soaking machine which keeps the liquid level in the imbibition tank constant.

Insert the cotton flap in the imbibition tank so that it is completely immersed. Leave the cotton flap in contact with the solution until it is completely soaked therewith.

### Drying

Slide the cotton flap through the two press rollers positioned at a distance of 1.5 mm. ± 07 mm.

Place the soaked and pressed cotton flap on the conveyor belt. When leaving the machine, the flap is portioned and placed inside mats and inserted in an oven at 50°C for 1.30 hrs, then at 40°C for 1hr and then at 30°C for 7.5hrs or until completely dry.

The final product has a content of Fe(III) ions equal to 15-35% by weight for each 2.5 g of hemostatic cotton.

### EXAMPLE 2 - TESTS WITH OTHER SALTS

Before reaching the formula object of the invention various other combinations of ferric and ferrous Salts with other potentially coagulating Salts were tested. Below are briefly reported the combinations of Salts tested and the results obtained also in comparison with Ferric Chloride.

| **SUBSTANCE** | **CONC. TEST (% by weight)** | **RESULTS** |
|---|---|---|
| Ferric Chloride (FeCl₃ · 6H₂O) | 5.00% | coagulates |
| Ammonium iron citrate Brown + Calcium chloride (CaCl₂ ·6H₂O) | 5.00% + 1.50% | does not coagulate |
| Iron sulphate heptahydrate (FeSO₄· 7H₂O) + Calcium chloride (CaCl₂. 6H₂O) | 2.50% + 1.50% | it was not possible to carry out the tests as the solution forms gypsum |
| Iron ammonium citrate (green) + Rock Alum | 1.9% + 0.63% | does not coagulate |
| Iron ammonium citrate (brown) + Rock Alum | 1.9% + 0.63% | coagulates |
| Iron ammonium citrate (red) + Rock Alum | 1.9% + 0.63% | coagulates |
| Iron ammonium citrate (red) + Iron sulphate heptahydrate (FeSO₄· 7H₂O) | 1.9% + 0.63% | little coagulates |
| Iron sulphate heptahydrate (FeSO₄· 7H₂O) + Rock alum | 5.3% | Does not coagulate |
| | 1.9% | |

### EXAMPLE 3 - VARIATION OF THE CONCENTRATION ACCORDING TO THE PRESSURE APPLIED ON THE COTTON FLAP

In the experimental phase it was assessed how the difference in pressure applied by the rollers on the flap allows reaching different concentrations according to the final topical application.

For example, the concentration of Salts to ensure effective coagulating activity in epistaxis must be 21% ± 3.5% while that for superficial abrasions due to rubbing must be 8% ± 2%; where % refer to percentages by weight with respect to each 100 g of finished product.

Using the press rollers of the invention, the distance to obtain concentrations at 21% must be 1.5 mm. ± 07 mm.

With the same concentration of wetting solution, in order to obtain a final concentration of 8% the rollers must be positioned 0.6 ± 0.3 mm.

### EXAMPLE 4

To verify the correct concentrations of Salts on the surface, an analytical method was developed which made it possible to verify how the process guarantees homogeneity and reproducibility of concentration in every part of the flap and repeatability.

### FAC titration

The method for the determination of Fe (3+) is based on iodometric titration.

Iodometric titrations fall within the broad field of oxydimetric titrations in which the oxidizing or reducing property is used.

Iodometry is based on the oxidation of iodide into elemental iodine according to the semi-reaction: 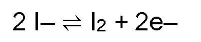
for which the normal reduction potential E° = - 0.53 V
Iodometry is used in the determination of oxidizing agents and is carried out in two stages:

### First stage:

In the first stage, the reaction takes place between iodide and an oxidizing agent such as permanganate, dichromate, peroxides, etc. according to the general scheme:

KI + oxidizing agent → I₂ + reduced state

In this case, the oxidizing agent is Fe (3+):

2 KI + 2 Fe (3+) → I₂ + 2 Fe (2+) + 2K(+)

In the reaction, iodine went from oxidation number -1 to oxidation number zero while iron went from oxidation number +3 to oxidation number +2.

### Second stage:

In the second stage, the iodine obtained in the first stage is titrated with a sodium thiosulfate solution which is transformed into tetrathionate.

The final point of the titration is highlighted by a specific indicator or the starch solution that must be added just before the final point, that is when the yellow-brown colour of the iodine has become barely visible. The solution turns blue as the amylose contained in the starch solution forms a blue-coloured adsorption compound.

### Analytical method: lodometric titration of the FAC

Transfer about 1 g of carefully weighed FAC to a 250-ml flask, dissolve with 25 ml of water and 5 ml of 37% HCl. Add 4 g of KI and wait, protecting from light, for 15 min.

Add 100 ml of water and titrate the released I₂ with 0.1N sodium thiosulfate using a starch solution as an indicator. Test with white on the bench.

1 ml of 0.1N sodium thiosulfate is equivalent to 5.585 mg of Fe.

Check the method at the concentration level of the two components in the finished product formulation.

### Considering:

| MP CODE | SUBSTANCE | PM / PA | QUANTITY | [IONS] |
|---|---|---|---|---|
| MP0A0448 | Ferric ammonium citrate | 279.99 / 55.85 | 5.3 g / 100 ml | 1.06 g / 100 ml |

And that 1 ml of 0.1N sodium thiosulfate is equivalent to 5.585 mg of Fe, the 5.3% FAC solution must be diluted 10 times in order to be titrated with a volume of around 20 ml.

### EXAMPLE 5 - In vitro assessment of cytotoxicity

The cytotoxic effect was assessed *in vitro* thanks to the use of murine fibroblasts (NIH3T3L6 fibroblasts from Leibniz-Institute DSMZ-Deutsche Sammlung von Mikroorganismen und Zellkulturen GmbH; Brunswick, Germany) and, once the cells were propagated, we prepared the following experimental groups:
- control (NIH3T3L6 cells without any treatment);
- iron chloride 5% (NIH3T3L6 cells incubated 30" with 100µL of iron chloride 5%);
- iron ammonium citrate 5.3% + Rock alum 1.7% (NIH3T3L6 cells incubated 30" with 100µL of iron ammonium citrate 5.3% + Rock alum 1.7%);
- iron ammonium citrate 5.3% (NIH3T3L6 cells incubated 30" with 100µL of iron ammonium citrate 5.3%);
- Rock alum 1.7% (NIH3T3L6 cells incubated 30" with 100µL of rock alum 1.7%).

The incubation time of 30 seconds was determined as the incubation of murine fibroblasts in 5% iron chloride for a time greater than 45"-50" causes complete cell death.

The in vitro cytotoxicity analysis thanks to the evaluation of the trypan blue allowed us to obtain the percentage of viable cells in each experimental group. In particular, we observed that the iron chloride 5% causes a significant cell death compared to both the control cells, but also compared to the formulation iron ammonium citrate 5.3% + Rock alum 1.7% or to the single component solutions of the new formulation, underlining how the formulation iron ammonium citrate 5.3% + Rock alum 1.7% has a significantly lower cytotoxic effect than iron chloride 5%.

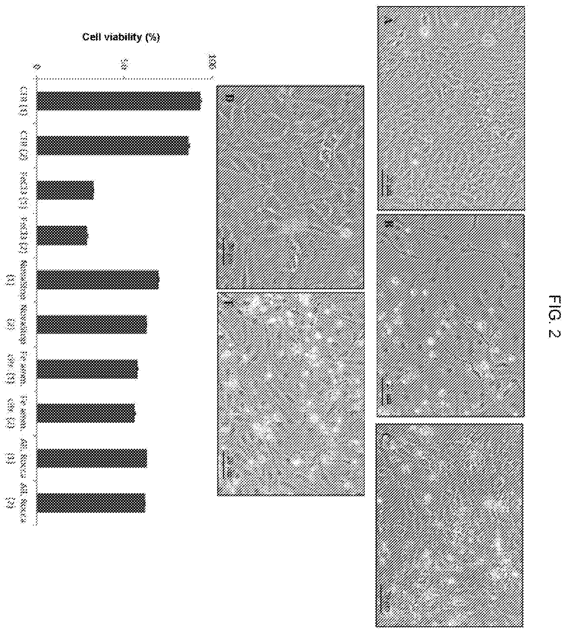

In the photomicrographs of fig 2 different experimental groups observed under the inverted microscope and the summary graph of cell viability can be observed.

### EXAMPLE 6 - Clinical study of the hemostatic activity of the hemostatic cotton of the invention (EMOSTOP)

### Patients enrolled

15 cases of nosebleeds,
22 ongoing cases of phlebological surgery,
8 cases of surgery for haemorrhoidal pathology.
5 cases of haemorrhage on phlebostatic ulcers.
2 cases of varicorrhagia with healthy skin.

### RESULTS

In all the tests the time for the arrest of the bleeding took from a few moments, to a maximum of 15 seconds, depending on the extent of the bleeding and the site. The hemostatic activity was produced with the contact of the product and with a slight compression on the tissue. This compression is already carried out autonomously when EMOSTOP is placed in a small cavity, such as the nasal nostril or a small surgical wound, thanks to the imbibition of the cotton and the subsequent increase in the volume thereof.

Once the arrest of the bleeding is obtained, it may be necessary to wait for at least 2 minutes, in case of more copious bleeding or site where the product cannot reach a direct contact with the bleeding site as the protein precipitation process must extend from the surface of the cotton to the bleeding area: since the first part of the clot remains adhered to the tissue, when it is removed, bleeding might resume.

The application of the product was always asymptomatic and did not alter the skin or mucous membranes surrounding the treated area, even when left in place for 7 days. When positioned on a trophic lesion, bleeding after curettage, on reassessment after about 4 days it was observed that the tissue was no longer soaked with liquid but containing coagulated blood and well detached from the site where the device had been positioned and therefore without relapse of bleeding upon removal thereof.

### CONCLUSIONS

EMOSTOP has excellent capability of bleeding arrest in extremely short times, with a slight compression of the product, without any alteration of the skin or mucous membranes on which it is applied.

### EXAMPLE 7 - Preparation of a hemostatic powder according to the present invention

Insert in a turbo emulsifier (AXOMIX 360) purified water at room temperature starting the mixing of the blades at a blade speed of 40 RPM.

Insert the following raw materials in the following order respecting the complete dissolution of the previous raw material.

### FAC

### ALUM

### ARGININE

### INTEGRINE-60 (Low molecular weight oligopeptide from pure rice proteins)

Once all the ingredients have been completely dispersed, proceed with the addition of hydrophobic colloidal silica (AEROSIL^{®} R812S, Silanamine, 1,1,1 -trimethyl-N-(trimethylsinyl)-hydrolysis products), start the turbine and the mixing of the blades at maximum speed until complete incorporation of the solution.

The ingredients can be modulated according to the pathology for which the powder is intended:
A) SUPERFICIAL ABRASIONS

| INGREDIENT | % w/w | Quantity in kg |
|---|---|---|
| FAC | 5.3 | 5.3 |
| ALUM | 1.7 | 1.7 |
| WATER | 100 | 100 |
| AEROSIL R812S | 6 | 6.0 |

B) VARICOSE ULCERS

| INGREDIENT | % w/w | Quantity in kg |
|---|---|---|
| FAC | 5.3 | 5.3 |
| ALUM | 1.7 | 1.7 |
| ARGININE | 1 | 1.0 |
| INTEGRINE-60 | 2 | 2.0 |
| WATER | 100 | 100 |
| AEROSIL R812S | 6 | 6.0 |

C) BEDSORES

| INGREDIENT | % w/w | Quantity in kg |
|---|---|---|
| FAC | 5.3 | 5.3 |
| ALUM | 1.7 | 1.7 |
| ARGININE | 3 | 3.0 |
| INTEGRINE | 2 | 2.0 |
| WATER | 100 | 100 |
| AEROSIL R812S | 6 | 6.0 |

D) DIABETIC FOOT

| MP DESCRIPTION | % w/w | Quantity in kg |
|---|---|---|
| FAC | 5.3 | 5.3 |
| ALUM | 1.7 | 1.7 |
| ARGININE | 5 | 5.0 |
| INTEGRINE | 2 | 2.0 |
| WATER | 100 | 100 |
| AEROSIL R812S | 6 | 6.0 |

| | | |
|---|---|---|
| where % w/w are with respect to the weight of the water. | | |

### EXAMPLE 8 - Clinical use of a hemostatic powder according to the present invention

The haemostatic powder of Example 7B was used to treat 3 Patients suffering from chronic ulcerative lesions due to chronic venous insufficiency CEAP stage V. PATIENT 1 (59-year-old male) suffering from long-standing post-thrombotic syndrome with both venous and lymphatic decompensation, with ulcer on the medial aspect of the lower 1/3 of the oval right leg sized about 2.30 cm, apparently not infected, with fibrinous bed, with abundant fibrinous serum secretion. The lesion was treated with cleansing with antiseptic, surgical curettage to remove excess fibrin, dressing with the hemostatic powder of the invention which stopped the bleeding caused by toileting, absorbed the excess exudate and avoided infection. The dressing was repeated every 2 days for 3 weeks, the residue of the previous dressing was always removed with ease, leaving the lesion well cleansed and with less fibrin formation, thus reducing the need for curettage, the edges of the ulcer did not present alteration due to the dressing. The patient tolerated the dressing well. At the end of the observation period, the lesion showed a notable reduction in exudation, a well granulating bed which allowed to set up an advanced dressing with cicatrising indication.

PATIENT 2 (56-year-old female) suffering from post-thrombotic syndrome for years, with recurrent ulcer in the anteromedial aspect of the left leg of irregular shape of about 2.20 cm on indurate cellulite. The same dressing protocol was applied as for the previous patient. Also in this case, in addition to the reduction of the exudate, which previously used to damage the surrounding tissues, a lesion with no more exudate, with a granular bed, of greatly reduced dimensions, was obtained.

PATIENT 3 (76-year-old male) suffering from chronic venous insufficiency in diabetic, with multiple ulcers on the anterior medial aspect of the left leg, about 5-8 cm in size each in dystrophic periwound skin, strongly painful, with abundant exudate, with a fibrinous haemorrhagic bed. The aforementioned protocol immediately resulted in a significant reduction in symptoms, absence of exudation with a reduction of the lesions to less than 50% of the extent such that they appeared well granulating.

In this experience, the hemostatic powder according to the present invention has therefore proved to be an excellent aid in the treatment of trophic lesions, both for its ability to maintain adequate humidity on the lesions, asepticity, stimulate cicatrisation and without altering the trophism of the surrounding skin even in the present of difficult situations.

## Claims

1. A hemostatic agent comprising or consisting of ferric ammonium citrate (FAC) and potassium alum (rock alum) wherein ferric ammonium citrate (FAC) is brown or red.

2. The hemostatic agent according to claim 1, wherein the Fe(lll) salt is present in a proportion by weight of 4:1-2:1 with respect to the Al(III) salt, preferably in a proportion of 3:1.

3. A hemostatic composition in a form adapted for topical application comprising the hemostatic agent according to any of claims 1-2.

4. The composition according to claim 3, in the form of powder, spray solution, gel, ointment or cream, or gauze or plaster.

5. The composition according to any of claims 3-4 further comprising one or more ingredients selected from arginine, modulator of Filaggrin and/or Fibronectin and/or Elastin, aloe, Silver ions, hyaluronic acid and Tamarindus Indica.

6. The composition according to any of claims 3-5 in the form of a powder and comprising as excipient/substrate hydrophobic colloidal silica.

7. The composition according to claim 6 comprising:
FAC 4-6% w/w; more preferably 5.0-5.5% w/w;
alum 1-2% w/w; more preferably 1.5-2.0% w/w;
silica 2-8% w/w; more preferably 5-7% w/w;
arginine 0-5% w/w; if present, more preferably 1-5% w/w;
integrine-60 0-5% w/w; if present more preferably 1.5-2.0% w/w;
purified water 100%;
where the aforesaid % w/w are meant with respect to the weight of the purified water.

8. A hemostatic cotton consisting of carded cotton and/or water treated cotton comprising a hemostatic agent according to any of claims 1-2.

9. A hemostatic cotton according to claim 8 wherein Fe(lll) is present in an amount equal to 15-40 g of Fe/100 g, preferably 20-40 g of Fe/100 g, or preferably 25-35 g of Fe/100 g.

10. The hemostatic agent according to any of claims 1-2 for medical use, in particular for use in the treatment of bleeding phenomena.

11. Process for preparing a hemostatic cotton according to any one of claims 8-9 comprising:
- preparation of a wetting aqueous solution comprising ferric ammonium citrate (FAC) and potassium alum (rock alum);
- imbibition of a cotton flap with the wetting solution;
- drying.

## Patentansprüche

1. Hämostatisches Mittel, umfassend oder bestehend aus Ammoniumeisen(III)-Citrat (FAC, ferric ammonium citrate) und Kaliumalaun (Steinalaun), wobei das Ammoniumeisen(III)-Citrat (FAC) braun oder rot ist.

2. Hämostatisches Mittel nach Anspruch 1, wobei das Fe(III)-Salz in einem Gewichtsanteil von 4 : 1-2 : 1 in Bezug auf das Al(III)-Salz, vorzugsweise in einem Anteil von 3 : 1, vorhanden ist.

3. Hämostatische Zusammensetzung in einer Form, die zur topischen Anwendung geeignet ist, umfassend das hämostatische Mittel nach einem der Ansprüche 1-2.

4. Zusammensetzung nach Anspruch 3 in Form von Pulver, Sprühlösung, Gel, Salbe oder Creme oder Gaze oder Pflaster.

5. Zusammensetzung nach einem der Ansprüche 3-4, ferner umfassend einen oder mehrere Inhaltsstoffe, ausgewählt aus Arginin, Modulator von Filaggrin und/oder Fibronektin und/oder Elastin, Aloe, Silberionen, Hyaluronsäure und Tamarindus indica.

6. Zusammensetzung nach einem der Ansprüche 3-5 in der Form eines Pulvers, das als Hilfsstoff/Substrat hydrophobe kolloidale Kieselerde umfasst.

7. Zusammensetzung nach Anspruch 6, umfassend:
FAC 4-6 Gew.-%; bevorzugter 5,0-5,5 Gew.-%;
Alaun 1-2 Gew.-%; bevorzugter 1,5-2,0 Gew.-%;
Kieselerde 2-8 Gew.-%; bevorzugter 5-7 Gew.-%;
Arginin 0-5 Gew.-%; falls vorhanden, bevorzugter 1-5 Gew.-%;
Integrin-60 0-5 Gew.-%; falls vorhanden, bevorzugter 1,5-2,0 Gew.-%; gereinigtes Wasser 100 %,
wobei sich die zuvor erwähnten Gew.-% auf das Gewicht des gereinigten Wassers beziehen.

8. Hämostatische Watte, die aus kardierter Baumwolle und/oder wasserbehandelter Baumwolle besteht, umfassend ein hämostatisches Mittel nach einem der Ansprüche 1-2.

9. Hämostatische Watte nach Anspruch 8,
wobei Fe(III) in einer Menge von 15-40 g Fe/100 g, vorzugsweise 20-40 g Fe/100 g oder vorzugsweise 25-35 g Fe/100 g, vorhanden ist.

10. Hämostatisches Mittel nach einem der Ansprüche 1-2 zur medizinischen Verwendung, insbesondere zur Verwendung bei der Behandlung von Blutungserscheinungen.

11. Verfahren zur Herstellung einer hämostatischen Watte nach einem der Ansprüche 8-9, umfassend:
- Herstellen einer wässrigen Netzmittellösung, umfassend Ammoniumeisen(III)-Citrat (FAC) und Kaliumalaun (Steinalaun);
- Tränken eines Watteumschlags mit der Netzmittellösung;
- Trocknen.

## Revendications

1. Agent hémostatique comprenant ou consistant en citrate d'ammonium ferrique (ferric ammonium citrate, FAC) et alun de potassium (alun de roche), dans lequel le citrate d'ammonium ferrique (FAC) est brun ou rouge.

2. Agent hémostatique selon la revendication 1, dans lequel le sel de Fe(III) est présent dans une proportion en poids de 4:1 à 2:1 par rapport au sel d'Al(III), de préférence dans une proportion de 3:1.

3. Composition hémostatique sous une forme adaptée à une application topique comprenant l'agent hémostatique selon l'une quelconque des revendications 1 à 2.

4. Composition selon la revendication 3, sous forme de poudre, de solution de pulvérisation, de gel, d'onguent ou de crème, ou de gaze ou de plâtre.

5. Composition selon l'une quelconque des revendications 3 à 4, comprenant en outre un ou plusieurs ingrédients choisis parmi l'arginine, le modulateur de la filaggrine et/ou fibronectine et/ou élastine, aloès, ions d'argent, acide hyaluronique et Tamarindus indica.

6. Composition selon l'une quelconque des revendications 3 à 5, sous la forme d'une poudre et comprenant comme excipient/substrat de la silice colloïdale hydrophobe.

7. Composition selon la revendication 6 comprenant :
FAC 4 à 6 % p/p ; plus préférablement 5,0 à 5,5 % p/p ;
alun 1 à 2 % p/p ; plus préférablement 1,5 à 2,0 % p/p ;
silice 2 à 8 % p/p ; plus préférablement 5 à 7 % p/p ;
arginine 0 à 5 % p/p ; si présent, plus préférablement 1 à 5 % p/p ;
intégrine-60 0 à 5 % p/p ; si présent plus préférablement 1,5 à 2,0 % p/p ; eau purifiée 100 %,
où les % p/p précités sont entendus par rapport au poids de l'eau purifiée.

8. Coton hémostatique constitué de coton cardé et/ou de coton traité à l'eau comprenant un agent hémostatique selon l'une quelconque des revendications 1 à 2.

9. Coton hémostatique selon la revendication 8,
dans lequel Fe(lll) est présent en une quantité égale à 15 à 40 g de Fe/100 g, de préférence 20 à 40 g de Fe/100 g, ou de préférence 25 à 35 g de Fe/100 g.

10. Agent hémostatique selon l'une quelconque des revendications 1 à 2 à usage médical, en particulier pour une utilisation dans le traitement des phénomènes hémorragiques.

11. Procédé de préparation d'un coton hémostatique, selon l'une quelconque des revendications 8 à 9 comprenant :
- préparation d'une solution aqueuse mouillante comprenant du citrate d'ammonium ferrique (FAC) et de l'alun de potassium (alun de roche) ;
- imbibition d'un rabat de coton avec la solution mouillante ;
- séchage.
